# EUROPÄISCHE PATENTSCHRIFT

(11) **EP 1 113 757 B1**
(45) Veröffentlichungstag und Bekanntmachung des Hinweises auf die Patenterteilung: **02.06.2004**
(21) Anmeldenummer: 98942440.3
(22) Anmeldetag: 17.09.1998
(51) Int. Cl.: A61B 17/58

(54) **REPOSITIONSINSTRUMENT ZUR FIXATION VON KNOCHENFRAKTUREN**
REPOSITIONING INSTRUMENT FOR THE FIXATION OF BONE FRACTURES
INSTRUMENT DE REDUCTION POUR LA FIXATION DE FRACTURES OSSEUSES

(43) Veröffentlichungstag der Anmeldung: 11.07.2001
(73) Patentinhaber: SYNTHES AG Chur, 7002 Chur (CH)
(72) Erfinder: HEHLI, Markus, CH-7276 Frauenkirch (CH); AMBÜHL, Ruedi, CH-7477 Filisur (CH); DELL'OCA, Alberto, Fernandez, 620/801 Montevideo (UY)
(74) Vertreter: Lusuardi, Werther Giovanni, Dr.
(86) Internationale Anmeldenummer: PCT/CH1998/000399
(87) Internationale Veröffentlichungsnummer: WO 2000/016709

(56) Entgegenhaltungen:
- CH-A- 607 915
- FR-A- 541 854
- FR-A- 1 169 404
- US-A- 2 631 585
- US-A- 3 114 367
- US-A- 5 312 403
- US-A- 5 645 548

## Beschreibung

Die Erfindung bezieht sich auf eine Vorrichtung zur externen Fixation von Knochenfrakturen gemäss dem Oberbegriff des unabhängigen Patentanspruchs 1.

Zur Fixierung von Knochen oder Knochenfragmenten stehen dem Chirurgen neben herkömmlichen Fixateur externe, bei welchen die Knochen oder Knochenfragmente mittels Schrauben oder Nägeln fixiert werden, auch Vorrichtungen zur Verfügung, wo die Knochen oder Knochenfragmente mittels Knochenklammern fixiert werden. Der Vorteil solcher Klammerfixationssysteme liegt in der geringeren Infektionsgefahr, die durch das Eindringen von Keimen entlang der Nägel oder Schrauben bis in den Knochen hervorgerufen wird, und in der ebenfalls geringeren Störung der Durchblutung des Knochens, welche durch das Einbringen von Nägeln oder Schrauben erheblich gestört wird.

Eine Vorrichtung zur Fixierung von Knochen oder Knochenfragmenten ist aus der FR 1 169 404 MONTEZIN bekannt. Diese bekannte Vorrichtung umfasst zwei oder mehrere zangenartige Instrumente zur Feststellung der Knochenfragmente. Am hinteren Ende eines der Zangenhebel ist eine Stange mit Zacken angebracht, so dass ein weiterer Zacken am hinteren Ende des anderen Zangenhebels mit einem der Zacken der Stange in Eingriff bringbar und arretierbar ist, wodurch ein Knochenfragment zwischen den Spitzen des zangenartigen Instrumentes lösbar festgeklemmt werden kann. Ferner ist verschiebbar an einem der Zangenhebel ein Klemmteil angebracht, wodurch das Instrument mittels einer Gewindestange und einem weiteren Klemmteil mit einem Längsstab verbindbar ist. An diesem Längsstab sind auf die gleiche Weise zwei oder mehrere der zangenartigen Instrumente anbringbar. Nachteilig an dieser Ausführung ist die verringerte Stabilität der Vorrichtung durch die Verschiebbarkeit und den komplizierten Aufbau des aus zwei Klemmteilen und einer Gewindestange bestehenden Verbindungsmittels.

Eine Knochenfasszange zum Festklammern eines Knochens oder Knochenfragmentes ist aus der CH 607 915 MATHYS bekannt. Diese bekannte Knochenfasszange umfasst zwei Zangenhebel, welche mittels einer Gewindestange relativ zueinander feststellbar sind. Diese Gewindestange ist am einen Zangenhebel schwenkbar gelagert und wird am anderen Zangenhebel in einer den Zangenhebel durchdringenden Bohrung aufgenommen. Festgestellt werden die Zangenhebel mittels einer Mutter, welche über die Gewindestange schraubbar ist. Nachteilig an dieser Knochenfasszange ist, dass keine speziellen Mittel zur Verbindung einer Knochenfasszange mit mindestens einer weiteren Knochenfasszange vorgesehen sind, so dass eine solche Verbindung bei Bedarf mindestens an einem der Zangenhebel angebracht werden müsste.

Aus der EP 0 457 017 FRIGG ist ein Klammerfixationssystem bekannt, bei welchem der Knochen durch die Spitzen einer Knochenfasszange an Stelle einer Schraube oder eines Nagels gefasst wird. Das hat den Vorteil, dass der Knochen nicht durchbohrt werden muss und dadurch keine Hitzenekrose hervorgerufen wird und auch die Durchblutung nur in minimalster Weise gestört wird. Mehrere solche Knochenfasszangen können analog zu Schrauben oder Nägeln extern miteinander verbunden werden. Ein Nachteil dieser bekannten Knochenfasszange liegt darin, dass sich der zur Blockierung der Zangenspitzen dienende Zangenverschluss und der Verbindungsstift, der eine Befestigung der Knochenfasszange innerhalb eines externen Knochenfixationssystems gestattet, im Drehgelenk der Knochenfasszange befinden, wodurch ein sehr voluminöses und kompliziertes Drehgelenk entsteht.

US-A-3 114 367 (CARPENTER) offenbart eine Vorrichtung, die die Merkmale des Oberbegriffes des Anspruchs 1 aufweist.

Hier will die Erfindung Abhilfe schaffen. Der Erfindung liegt die Aufgabe zugrunde, eine Verbindung mehrerer Repositionszangen innerhalb einer stabilen Vorrichtung zur Fixation von Knochenfrakturen zu schaffen, wobei diese Vorrichtung einfache, manuell bedienbare Repositionszange umfasst, welche eine nicht-invasive, temporäre Fixation von Knochenfrakturen ermöglicht.

Die Erfindung löst die gestellte Aufgabe mit einer Vorrichtung zur externen Fixation von Knochenfrakturen, welche die Merkmale des Anspruchs 1 aufweist.

Weitere vorteilhafte Ausgestaltungen der Erfindung sind in den abhängigen Ansprüchen gekennzeichnet.

Die erfindungsgemässe_Vorrichtung zur externen Fixation von Knochenfrakturen umfasst mindestens eine Repositionszange, mindestens einen Längsträger und ein den Verbindungsbolzen der Repositionszange und einen Längsträger gegeneinander drehbar und in lösbarer Weise verbindendes Klemmelement. Das Klemmelement besteht aus zwei gegeneinander um eine Drehachse drehbaren Klemmteilen, wobei jedes der Klemmteile mit einer durchgehenden zylindrischen Bohrung versehen ist. Diese Bohrungen können dem Querschnitt des Verbindungsbolzen und des Längsträgers entsprechend einen kreiszylindrischen Querschnitt aufweisen. Die Drehachse, um welche die beiden Klemmteile gegeneinander schwenkbar sind, steht senkrecht auf der durch die beiden Längsachsen der Bohrungen aufgespannten Ebene. Mittels einer konzentrisch zur Drehachse verlaufenden, durch die Bohrungen in den Klemmteilen durchgehenden Spannschraube können die beiden mit Schlitzen versehenen Klemmteile zusammenpresst und gleichzeitig gegeneinander gepresst werden, wodurch der im einen Klemmteil eingespannte Verbindungsbolzen der Repositionszange und der in die Bohrung des andere Klemmteiles eingeführte Längsträger in den Klemmteilen blockiert und zugleich die gegeneinander verdrehbaren Klemmteile in einer gewählten Position gegeneinander fixiert werden. Zur besseren Fixierung der beiden Klemmteile gegen Verdrehung sind die beiden miteinander in Berührung stehenden Stirnflächen der Klemmteile mit ineinander eingreifenden Verzahnungen versehen.

Die Repositionszange umfasst zwei durch ein Verbindungsmittel um eine Drehachse schwenkbar miteinander verbundene Zangenhebel mit Klemmspitzen an den zum Greifen des Knochens bestimmten Enden der Zangenhebel, ein Feststellmittel, durch welches die Zangenhebel in lösbarer Weise gegeneinander gepresst werden können und auch feststellbar sind, und einen Verbindungsbolzen, mittels welchem eine Verbindung zu einem weiteren Element innerhalb einer Repositionsvorrichtung herstellbar ist. Das Feststellmittel ist in einem Abstand y zur Drehachse angebracht während der Verbindungsbolzen in einem Abstand x zur Drehachse angebracht ist. Die Längsachse des Verbindungsbolzens liegt in der Schwenkebene der Zangenhebel.

Die Abstände x zwischen Drehachse und Verbindungsbolzen und y zwischen Drehachse und Feststellmittel können auch gleich gross sein. Der Abstand x wird jedoch vorzugsweise klein gewählt damit keine grossen Hebel zwischen den den Knochen fassenden Klemmspitzen und dem Verbindungsbolzen auftreten, da sonst grosse Drehmomente im Verbindungsbolzen auftreten können. Der Abstand y dagegen wird vorzugsweise so gross gewählt, dass durch die Klemmkraft der Repositionszange keine übermässig hohen Zugkräfte im Feststellmittel auftreten.

Als Verbindungsmittel, welches die beiden Zangenhebel schwenkbar miteinander verbindet, ist eine Schraube vorgesehen, welche in einen der Zangenhebel mittels eines Gewindes eingeschraubt wird und im anderen der Zangenhebel drehbar gelagert ist. Der Verbindungsbolzen weist einen kreiszylindrischen Querschnitt auf.

Als Feststellmittel dient eine mit dem einen Zangenhebel fest verbundene Feststellschraube und eine auf diese aufschraubbare Mutter. Dabei ist die Feststellschraube im anderen Zangenhebel mittels eines Langloches beim Öffnen oder Schliessen der Zangenhebel entlang dieses anderen Zangenhebels verschiebbar.

Zudem kann die Mutter an ihrem am Zangenhebel anliegenden Ende mit einem Konus versehen sein.

Ebenfalls möglich ist die Ausgestaltung des Feststellmittels mit einem am ersten Zangenhebel angebrachten in verschiedene Stellungen umschaltbaren Klemmmechanismus und einer an der Innenseite des zweiten Zangenhebels angebrachten mit Zacken versehenen Formstange. Der Klemmmechanismus greift lösbar in die Zacken der Formstange ein. Die Zacken sind so ausgebildet, dass der Klemmmechanismus beim Zusammenpressen der Zangenhebel selbsttätig in die Zacken an der Formstange einrastet. Zum Lösen der festgestellten Zange muss der Klemmmechanismus von Hand gelöst werden, was durch Umschalten des Klemmmechanismusses in eine Stellung, in welcher die Zacken an der Formstange freigegeben werden, erfolgt.

Die Spannkraft der Zangenhebel und die Position der Repositionszange innerhalb der Fixationsvorrichtung können durch den vom Drehgelenk der Repositionszange entfernten und vom Feststellmittel unabhängigen Verbindungsbolzen unabhängig voneinander eingestellt werden. Zudem ergibt sich durch die Separierung von Verbindungsmittel, Feststellmittel und Drehgelenk eine einfache und wenig voluminöse Gestaltung des Drehgelenkes.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Darstellung der Repositionszange;
Fig. 2 eine perspektivische Darstellung einer Ausführungsform der erfindungsgemässen Vorrichtung zur externen Fixation von Knochenfrakturen; und
Fig. 3 eine perspektivische Darstellung eines Klemmelementes gemäss einer Ausführungsform der erfindungsgemässen Vorrichtung zur externen Fixation von Knochenfrakturen.

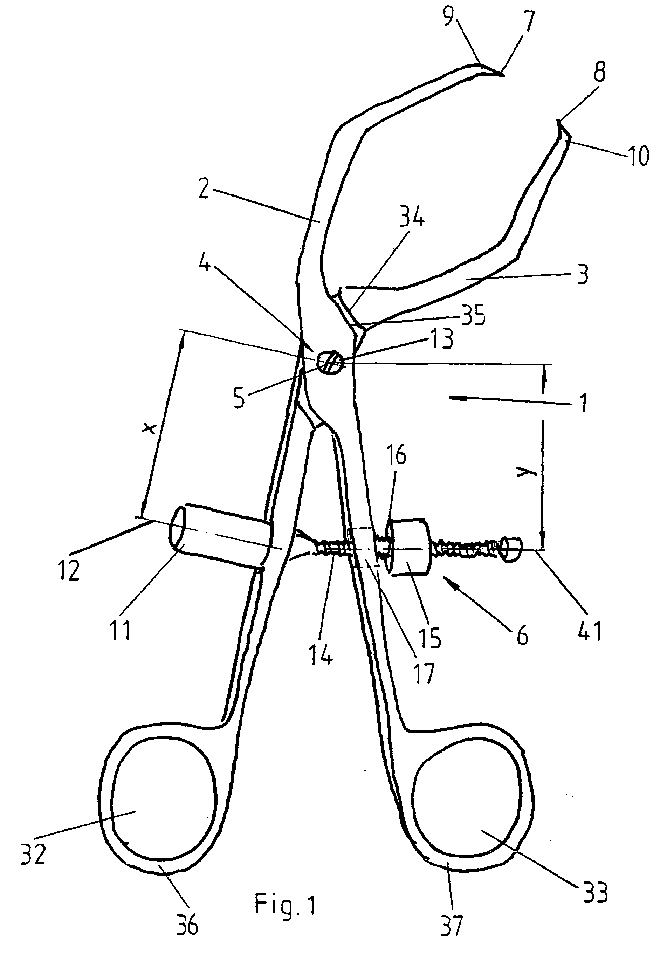

In Fig. 1 ist eine Ausführungsform der Repositionszange 1 dargestellt. Diese umfasst zwei Zangenhebel 2;3, welche um eine Drehachse 5 gegeneinander schwenkbar sind. Diese drehbare Verbindung der beiden Zangenhebel 2;3 wird durch eine Schraube 13, welche in einen der Zangenhebel 2 oder 3 mittels eines Gewindes eingeschraubt ist und im anderen Zangenhebel 2 oder 3 drehbar gelagert ist, hergestellt. Begrenzt wird die Drehbewegung beim Spreizen der Zangenhebel 2;3 durch an den Zangenhebeln 2;3 angebrachte Anschlagsschultern 34;35. An den zum Greifen eines Knochens bestimmten Enden 9;10 der Zangenhebel 2;3 sind scharfe Klemmspitzen 7;8 angebracht, während die zur Bedienung der Repositionszange 1 dienenden Enden 36;37 mit ringförmigen Fingeröffnungen 32;33 versehen sein können. Daneben umfasst die Repositionszange 1 ein Feststellmittel 6, welches in der gezeigten Ausführungsform durch eine an einen Zangenhebel 3 angebrachte Feststellschraube 14 und eine auf das Gewinde der Feststellschraube 14 schraubbare Mutter 15 ausgeführt ist. Die Feststellschraube 14 ist im Zangenhebel 2 in einem Langloch 17 verschiebbar gelagert und somit bei Schwenkbewegungen der Zangenhebel 2;3 entlang des Zangenhebels 2 verschieblich. Durch die Feststellschraube 14 und die Mutter 15 können die Zangenhebel 2;3 in einer Position lösbar so blockiert werden, dass sich die Zangenhebel 2;3 nicht weiter spreizen lassen. Bei einem zwischen den Klemmspitzen 7;8 eingespannten Knochen besteht auch die Möglichkeit mittels Feststellschraube 14 und Mutter 15 eine einmal aufgebrachte Einspannkraft aufrecht zu erhalten. Bei der hier gezeigten Ausführungsform der Repositionszange 1 ist die Feststellschraube 14 in einem Abstand y von der Drehachse 5 gegen die Enden 36;37 angebracht. Im weiteren umfasst die erfindungsgemässe Repositionszange 1 in der hier gezeigten Ausführungsform einen Verbindungsbolzen 11 mit einer Längsachse 12 und einem kreiszylindrischen Querschnitt mittels welchem die Repositionszange 1 an einem weiteren Element eines externen Fixateurs angebracht werden kann. Der Verbindungsbolzen 11 ist an einem der Zangenhebel 2;3 in einem Abstand x von der Drehachse 5 gegen die Enden 36;37 angebracht, wobei die Längsachse 12 in der Schwenkebene der Zangenhebel 2;3 liegt.

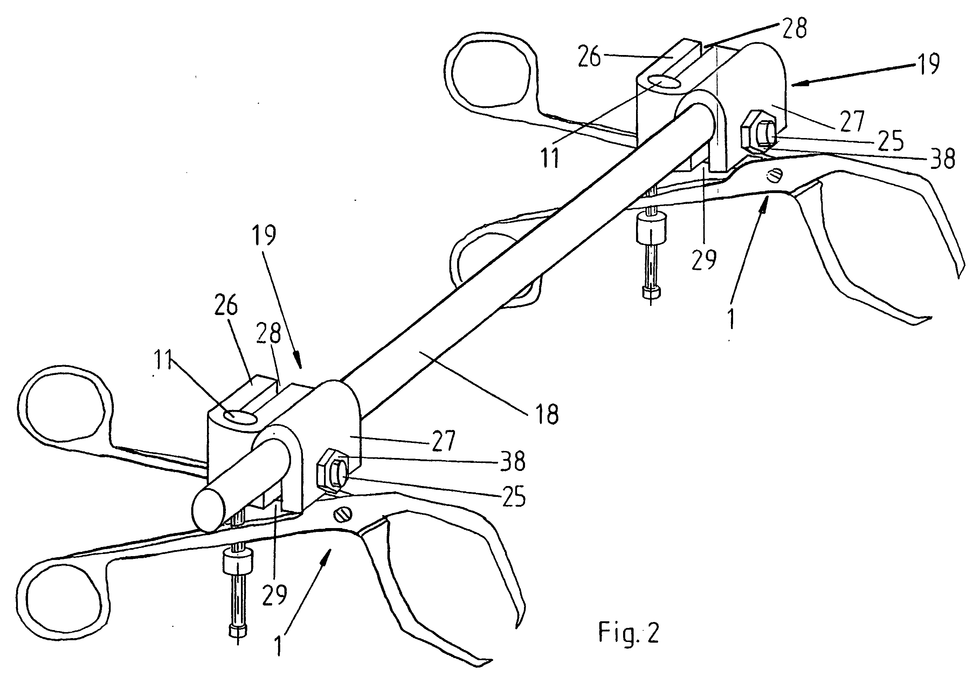

In Fig. 2 ist eine Ausführungsform der erfindungsgemässen Vorrichtung zur externen Fixation von Knochenfrakturen gezeigt. Die Vorrichtung umfasst zwei der in Fig. 1 dargestellten Repositionszangen, einen Längsträger 18 zur Verbindung der Repositionszangen und zwei Klemmelemente 19 mittels welcher die Repositionszangen 1 am Längsträger 18 befestigbar sind. Zur Zusammenstellung der hier gezeigten Ausführungsform der erfindungsgemässen Vorrichtung werden die an einem der Zangenhebel 2;3 fest angebrachten Verbindungsbolzen 11 in entsprechende Bohrungen in den einen Klemmteilen 26 der Klemmelemente 19 eingebracht während in die Bohrungen der anderen Klemmteile 27 der Längsträger 18 eingeführt wird. Zur Fixierung der Vorrichtung werden die Verbindungsbolzen 11 und der Längsträger 18 in den Bohrungen der Klemmteile 26;27, welche mit Schlitzen 28;29 versehen sind, mittels Spannschrauben 25, welche die Klemmteile 19 durchdringen, und an den mit Gewinden versehenen Enden der Spannschrauben 25 aufgeschraubten Muttern 38 festgeklemmt.

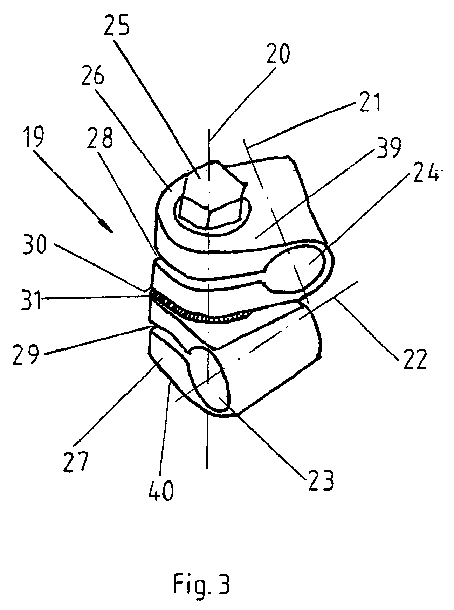

Fig. 3 zeigt eine Ausführungsform eines der zur erfindungsgemässen Vorrichtung gehörenden Klemmelementes 19. Das Klemmelement 19 besteht aus zwei separaten Klemmteilen 26;27, welche mit Bohrungen 23;24 mit den Längsachsen 21 und 22 durchgehend durchbohrt sind. Parallel zu den Stirnflächen 39;40 der Klemmteile 26;27 sind diese mit Schlitzen 28;29 ausgestattet, welche in die Bohrungen 23;34 münden. Dadurch können in die Bohrungen 23;24 der Klemmteile 26;27 eingespannte Verbindungsbolzen 11 von Repositionszangen 1 (Fig. 1 und 2) und Längsträger 18 (Fig. 2) durch Zusammenpressen der Klemmteile 26;27 in diesen fixiert werden. Durch Anziehen einer auf die Spannschraube 25, welche die Klemmteile 26;27 in entsprechenden Durchgangsbohrungen rechtwinklig zur einer durch die Längsachsen 22;23 der Bohrungen 23;24 aufgespannten Ebene durchdringt, aufschraubbaren Mutter 38 (Fig. 2) werden die Klemmteile 26;27 zusammengepresst und gleichzeitig an ihren sich gegenseitig berührenden Stirnflächen 30;31 gegeneinander gepresst. Die in die Bohrungen der Klemmteile 26;27 eingeführte Spannschraube 25 dient gleichzeitig auch als Drehachse 20, wodurch die beiden Klemmteite 26;27 in zueinander parallelen Ebenen um 360° rotierbar sind. Zur besseren Blockierung der gesamten erfindungsgemässen Vorrichtung sind die sich gegenseitig berührenden Stirnflächen 30;31 der Klemmteile 26;27 mit ineinander eingreifenden Verzahnungen versehen.

## Patentansprüche

1. Vorrichtung zur externen Fixation von Knochenfrakturen mit
A) mindestens zwei Zangen (1) zum Fassen von Knochen oder Knochenfragmenten, und
B) mindestens einem Längsträger (18),
wobei jede der Zangen (1)
a) zwei durch ein Verbindungsmittel (4) um eine Drehachse (5) schwenkbar miteinander verbundene Zangenhebel (2;3) zum Greifen eines Knochens; und
b) mindestens ein longitudinales Feststellmittel (6) mit einer Längsachse (41) umfasst, wobei die Zangenhebel (2;3) durch das Feststellmittel (6) in lösbarer Weise gegeneinander fixierbar sind; und jede der Zangen (1)
c) einen Verbindungsbolzen (11) mit einer Längsachse (12) umfasst, mittels welchem eine Verbindung mit einem weiteren Element innerhalb einer Repositionsvorrichtung herstellbar ist, wobei
d) die Längsachse (41) des Feststellmittels (6) in einem Abstand y zur Drehachse (5) angebracht ist; und
e) die Längsachse (12) des Verbindungsbolzens (11) in der Schwenkebene der Zangenhebel (2;3) liegt,
C) mindestens zwei, die Verbindungsbolzen (11) jeder Zange (1) und den mindestens einen Längsträger (18) gegeneinander drehbar und in lösbarer Weise verbindende Klemmelemente (19) umfasst,
**dadurch gekennzeichnet, dass**
D) jedes Klemmelement (19) zwei gegeneinander um eine Drehachse (20) drehbare Klemmteile (26;27) umfasst,
E) die beiden miteinander in Berührung stehenden Stirnflächen der Klemmteile (26;27) mit ineinander eingreifenden Verzahnungen (30;31) versehen sind; und
F) das Verbindungsmittel (4), welches die beiden Zangenhebel (2;3) schwenkbar miteinander verbindet, eine Schraube (13) ist, welche in einen der Zangenhebel (2;3) mittels eines Gewindes einschraubbar ist und im anderen der Zangenhebel (2;3) drehbar gelagert ist.

2. Vorrichtung nach Anspruch 1, **dadurch gekennzeichnet, dass** die Längsachse (12) des Verbindungsbolzens (11) in einem Abstand x zur Drehachse (5) angebracht ist.

3. Vorrichtung nach Anspruch 1 oder 2, **dadurch gekennzeichnet, dass** der Verbindungsbolzen (11) einen kreiszylindrischen Querschnitt hat.

4. Vorrichtung nach einem der Ansprüche 1 bis 3, **dadurch gekennzeichnet, dass** das Feststellmittel (6) eine mit dem einen Zangenhebel (3) verbundene Feststellschraube (14) und eine Mutter (15) umfasst, wobei die Feststellschraube (14) im anderen Zangenhebel (2) mittels eines Langloches (17) entlang des Zangenhebels (2) verschiebbar ist.

5. Vorrichtung nach einem der Ansprüche 1 bis 3, **dadurch gekennzeichnet, dass** das Feststellmittel (6) in der Art von Zahnstangen ausgebildet ist, die eine Haltung der Zangenhebel (2;3) in jeder Position ermöglichen.

6. Vorrichtung nach Anspruch 1 bis 3, **dadurch gekennzeichnet, dass** das Feststellmittel (6) am ersten Zangenhebel (3) einen in verschiedene Stellungen umschaltbaren Klemmmechanismus und an der Innenseite des zweiten Zangenhebels (2) eine mit Zacken versehene Formstange umfasst, wobei der Klemmmechanismus lösbar in die Zacken der Formstange eingreift.

7. Vorrichtung nach Anspruch 4, **dadurch gekennzeichnet, dass** die Mutter (15) an ihrem am Zangenhebel (2) anliegenden Ende (16) mit einem Konus versehen ist.

8. Vorrichtung nach einem der Ansprüche 1 bis 7, **dadurch gekennzeichnet, dass** jedes der Klemmteile (26;27) mit einer durchgehenden zylindrischen Bohrung (23;24) versehen ist, wobei die Bohrungen (23;24) die Längsachsen (21;22) aufweisen.

9. Vorrichtung nach Anspruch 8, **dadurch gekennzeichnet, dass** die Bohrungen (23;24) einen kreiszylindrischen Querschnitt aufweisen.

10. Vorrichtung nach einem der Ansprüche 1 bis 9, **dadurch gekennzeichnet, dass** die Drehachse (20) senkrecht auf der durch die beiden Längsachsen (21;22) aufgespannten Ebene steht.

11. Vorrichtung gemäss einem der Ansprüche 1 bis 10, **dadurch gekennzeichnet, dass** mittels einer konzentrisch zur Drehachse (20) verlaufenden, durch die Bohrungen (23;24) in den Klemmteilen (26;27) durchgehenden Spannschraube (25) die beiden mit Schlitzen (28;29) versehenen Klemmteile (26;27) zusammenpressbar sind und gleichzeitig die Klemmteile (26;27) gegeneinander gepresst werden.

## Claims

1. Device for externally fixate bone fractures comprising
A) at least two tongs (1) to seize bone or bone fragments, and
B) at least one elongated support (18),
whereby each of the tongs (1) comprises
a) two tongs levers (2;3) to seize a bone connected by a connecting means (4) so as to be mutually rotatable about an axis of rotation (5),
b) at least one longitudinal locking means (6) having a longitudinal axis (41), the tongs levers (2;3) being detachably affixed in mutual positions by the locking means (6); and each of the tongs (1) comprising
c) a connecting bolt (11) having a longitudinal axis (12) by means of which a connection can be implemented with a further component within a repositioning device; whereby
d) the longitudinal axis (41) of the locking means (6) is at a distance y form the axis of rotation (5), and
e) the longitudinal axis (12) of the connecting bolt (11) is situated in a plane of pivoting of the two tongs levers (2;3),
C) at least two clamps (19) detachably connecting in mutually rotating manner the connecting bolts (11) of each tong (1),
**characterised in that**
D) each clamp (19) comprises two clamp elements (26;27) mutually rotatable about an axis of rotation (20);
E) the two mutually abutting end faces of the clamp elements (26;27) are fitted with mutually engaging serrations (30;31); and
F) the connecting means (4) connecting the two tongs levers (2;3) to each other is a screw (13) which can be screwed into one of the tongs levers (2;3) fitted with thread and which is rotatably supported in the other tongs levers (2;3).

2. Device according to claim 1, **characterised in that** the longitudinal axis (12) of the connecting bolt (11) is at a distance x from the axis of rotation (5).

3. Device according to claim 1 or 2, **characterized in that** the cross-section of the connection bolt (11) is circular-cylindrical.

4. Device according to one of the claims 1 through 3, **characterized in that** the locking means (6) comprises a locking screw (14) connected to one (3) of the tongs levers and a nut (15), the locking screw (14) being displaceable in the other tongs lever (2) by means of an elongated slot (17) along the tongs lever (2).

5. Device according to one of the claims 1 through 3, **characterized in that** the locking means (6) is designed in the manner of a gear racks allowing locking the tongs levers (2, 3) in any position.

6. Device according to one of the claims 1 through 3, **characterized in that** the locking means (6) comprises, at the first tongs lever (3), a clamp switchable into different positions, and at the inside of the second tongs lever (2), a serrate shaped rod, the clamp detachably engaging the serrations of the shaped rod.

7. Device according to claim 4, **characterized in that** the nut (15) is fitted with a cone at its end (16) resting against the tongs lever (2).

8. Device according to one of the claims 1 through 7, **characterized in that** each clamp element (26, 27) is fitted with a continuous cylindrical borehole (23 , 24) comprising a longitudinal axis (21, 22).

9. Device according to claim 8, **characterized in that** the cross-sections of the boreholes (23, 24) are circular-cylindrical.

10. Device according to one of the claims 1 through 9, **characterized in that** the axis of rotation (20) is perpendicular to the plane subtended by the two longitudinal axes (21, 22).

11. Device according to one of the claims 1 through 10, **characterized in that** the two clamp elements (26, 27) fitted with slots (28, 29) can each be compressed by a tightening screw (25) passing concentrically with the axis of rotation (20) through the borehole (23, 24) in the clamp elements (26, 27) and **in that** simultaneously the clamp elements (26, 27) are forced against each other.

## Revendications

1. Dispositif pour la fixation externe de fractures osseuses avec
A) au moins deux pinces (1) pour saisir les os ou fragments d'os et
B) au moins un support longitudinal (18),
chacune des pinces (1)
a) comprenant deux branches de pince (2;3) reliées entre elles par un organe de liaison (4) et tournant autour d'un axe de rotation (5), destinées à saisir un os; et
b) au moins un organe de blocage longitudinal (6) avec un axe longitudinal (41), les branches de la pince (2;3) pouvant être bloquées de façon amovible l'une par rapport à l'autre à l'aide de l'organe de blocage (6); et chacune des pinces (1)
c) comprenant un goujon d'assemblage (11) avec un axe longitudinal (12) au moyen duquel on peut établir une liaison avec un autre élément au sein d'un dispositif de réduction,
d) l'axe longitudinal (41) de l'organe de blocage (6) étant placé à une distance y de l'axe de rotation (5); et
e) l'axe longitudinal (12) du goujon d'assemblage (11) se trouvant dans le plan de pivotement des branches de la pince (2;3),
C) comprenant au moins deux éléments de serrage (19) reliant de façon amovible et mobile entre eux les goujons d'assemblage (11) et le support longitudinal (18) minimum,
**caractérisé en ce que**
D) chaque élément de serrage (19) comprend deux pièces de serrage (26;27) mobiles l'une par rapport à l'autre autour d'un axe de rotation (20), **en ce que**
E) les deux surfaces frontales en contact des pièces de serrage (26;27) sont munies de dentures (30;31) s'imbriquant les unes dans les autres; et **en ce que**
F) l'organe de liaison (4) qui relie les deux branches de la pince (2;3) de façon mobile est une vis (13) qui peut se visser sur une des branches de la pince (2;3) à l'aide d'un filetage et qui est montée sur paliers orientables sur l'autre branche de la pince (2;3).

2. Dispositif selon la revendication 1, **caractérisé en ce que** l'axe longitudinal (12) du goujon d'assemblage (11) est placé à une distance x de l'axe de rotation (5).

3. Dispositif selon la revendication 1 ou 2, **caractérisé en ce que** le goujon d'assemblage (11) a une section cylindrique circulaire.

4. Dispositif selon une des revendications 1 à 3, **caractérisé en ce que** l'organe de blocage (6) comprend une vis de blocage (14) reliée à une branche de la pince (3) et un écrou (15), la vis de blocage (14) dans l'autre branche de la pince (2) étant mobile le long de la branche de la pince (2) au moyen d'un trou oblong (17).

5. Dispositif selon une des revendications 1 à 3, **caractérisé en ce que** l'organe de blocage (6) a la forme de crémaillères qui permettent de maintenir les branches de la pince (2;3) dans toutes les positions.

6. Dispositif selon une des revendications 1 à 3, **caractérisé en ce que** l'organe de blocage (6) comprend, sur la première branche de la pince (3), un mécanisme de serrage qui peut être mis dans différentes positions et, sur la face interne de la deuxième branche de la pince (2), une barre profilée munie de dents, le mécanisme de serrage s'enclenchant de façon amovible dans les dents de la barre profilée.

7. Dispositif selon la revendication 4, **caractérisé en ce que** l'écrou (15) est muni d'un cône sur son extrémité (16) appuyant contre la branche de la pince (2).

8. Dispositif selon une des revendications 1 à 7, **caractérisé en ce que** chacune des pièces de serrage (26;27) est munie d'un alésage cylindrique traversant (23;24), les alésages (23;24) possédant les axes longitudinaux (21;22).

9. Dispositif selon la revendication 8, **caractérisé en ce que** les alésages (23;24) présentent une section cylindrique circulaire.

10. Dispositif selon une des revendications 1 à 9, **caractérisé en ce que** l'axe de rotation (20) est perpendiculaire au plan formé par les deux axes longitudinaux (21;22).

11. Dispositif selon une des revendications 1 à 10, **caractérisé en ce qu'**un boulon de serrage (25) concentrique par rapport à l'axe de rotation (20) et traversant les alésages (23;24) dans les pièces de serrage (26;27) permet de resserrer les deux pièces de serrage (26;27) munies de fentes (28;29) tout en pressant les pièces de serrage (26;27) l'une contre l'autre.
